# EUROPÄISCHE PATENTANMELDUNG

(11) **EP 4 285 845 A1**
(43) Veröffentlichungstag der Anmeldung: **06.12.2023**
(21) Anmeldenummer: 23175955.6
(22) Anmeldetag: 30.05.2023
(51) Int. Cl.: A61B 17/15

(54) **CHIRURGISCHES INSTRUMENT**

(30) Priorität: 03.06.2022 DE 102022205694
(71) Anmelder: Aesculap AG, 78532 Tuttlingen (DE)
(72) Erfinder: Firmbach, Franz-Peter, 78576 Emmingen-Liptingen (DE); Anhorn, Svenja, 72535 Heroldstatt (DE)
(74) Vertreter: Patentanwälte Ruff, Wilhelm, Beier, Dauster & Partner mbB

(57) **Zusammenfassung**

Ein derartiges chirurgisches Instrument zur Verwendung bei einer Kniegelenkersatzoperation, aufweisend einen Grundkörper, welcher eingerichtet ist zur lösbaren Befestigung an einem Tibiaschnittblock zur Schnittführung an einer proximalen Tibia, einen ersten Taster und einen zweiten Taster, welche jeweils relativ zu dem Grundkörper beweglich an demselben gelagert und jeweils entlang einer Längsachse zwischen einem ersten Ende und einem zweiten Ende längserstreckt sind, wobei die ersten Enden jeweils zur Kontaktierung eines Tibiaplateaus der Tibia eingerichtet sind, ist bekannt.

Erfindungsgemäß ist der erste Taster relativ zu dem Grundkörper und dem zweiten Taster um eine erste Schwenkachse schwenkbeweglich an dem Grundkörper gelagert, und der zweite Taster ist relativ zu dem Grundkörper und dem ersten Taster um eine zweite Schwenkachse schwenkbeweglich an dem Grundkörper gelagert.

Verwendung bei einer Kniegelenkersatzoperation

## Beschreibung

Die Erfindung betrifft ein chirurgisches Instrument zur Verwendung bei einer Kniegelenkersatzoperation, aufweisend einen Grundkörper, welcher eingerichtet ist zur lösbaren Befestigung an einem Tibiaschnittblock zur Schnittführung an einer proximalen Tibia, einen ersten Taster und einen zweiten Taster, welche jeweils relativ zu dem Grundkörper beweglich an demselben gelagert und jeweils entlang einer Längsachse zwischen einem ersten Ende und einem zweiten Ende längserstreckt sind, wobei die ersten Enden jeweils zur Kontaktierung eines Tibiaplateaus der Tibia eingerichtet sind.

Bei einer Kniegelenkersatzoperation (englisch: Total Knee Arthroplasty (TKA)) werden abgenutzte oder anderweitig durch Krankheit oder einen Unfall beeinträchtigte Gelenkflächen des Femurs und/oder der Tibia durch künstliche Gelenkflächen einer Kniegelenkprothese ersetzt. Solche Kniegelenkprothesen umfassen üblicherweise eine Femurkomponente und eine Tibiakomponente. Die Femurkomponente wird am distalen Ende des Femurs implantiert. Die Tibiakomponente wird am proximalen Ende der Tibia implantiert.

Vor der Implantation der prothetischen Komponenten werden der distale Femur und die proximale Tibia reseziert. Hierfür bringt der Chirurg unterschiedliche Resektionsschnitte an und trennt Knochen- und/oder Knorpelmaterial von dem jeweiligen Knochen ab. Durch die Resektion wird der jeweilige Knochen in seiner Form an die aufzunehmende prothetische Komponente angepasst.

Die Resektion kann auf der Grundlage unterschiedlicher Konzepte ausgeführt werden. Ein Konzept zielt darauf ab, die Spannungen der Bänder des Knies bei der Gelenkbewegung ausgeglichen zu halten. Hierdurch soll eine bessere Funktion der Kniegelenkprothese gewährleistet werden. Dieses Konzept wird allgemein als "gap balancing" bezeichnet. Bei anderen Konzepten entfernt der Chirurg mittels der Resektion eine bestimmte Menge an Knochen- und/oder Knorpelmaterial. Solche Konzepte werden allgemein als "measured resection" bezeichnet. Die Ausrichtung der Resektionsschnitte in Bezug auf die Anatomie des Patienten bestimmt die spätere Ausrichtung der implantierten Komponenten und folglich auch die Orientierung der prothetischen Gelenkachsen. Die Ausrichtung der Resektionsschnitte ist daher von besonderer Bedeutung.

Bei der Ausrichtung der Resektionsschnitte werden in erster Linie drei Ansätze unterschieden: mechanisch, anatomisch und kinematisch. Bei der mechanischen Ausrichtung wird die proximale Tibia senkrecht zur Längsachse des Tibiaschafts reseziert. Die Resektion des distalen Femurs erfolgt entsprechend hieran angepasst. Erforderlichenfalls werden Bandentlastungen (englisch: ligament releases) durchgeführt. Bei der anatomischen Ausrichtung wird versucht, die Tibia in einem Varus-Winkel von 3° zu resezieren. Die Femurresektion und Bandentlastungen werden mit dem Ziel einer geraden Hüft-Knie-Knöchel-Achse des Beins durchgeführt. Das Ziel der kinematischen Ausrichtung (englisch: kinematic alignment, nachfolgend abgekürzt als KA) besteht darin, die künstlichen Gelenkflächen der prothetischen Komponenten auf dem Niveau der präarthritischen, defektfreien natürlichen Gelenkflächen zu implantieren. Klinische Studien haben gezeigt, dass mittels KA oftmals eine verbesserte Patientenzufriedenheit erreicht werden kann, da die Funktion des künstlichen Kniegelenks als eher natürlich empfunden wird.

Vor diesem Hintergrund besteht ein grundsätzlicher Bedarf nach möglichst präzisen, einfach verwendbaren und kosteneffizienten chirurgischen Instrumenten zur Umsetzung des KA.

Die vorliegende Erfindung befasst sich mit einem solchen chirurgischen Instrument, im Speziellen mit einem chirurgischen Instrument zur Ausrichtung der Tibiaresektion. Solche Instrumente werden auch als (tibiales) Ausrichtinstrument oder tibial resection guide device bezeichnet.

Ein derartiges chirurgisches Instrument ist aus der WO 2020/049421 A1 bekannt und weist einen Grundkörper und zwei Taster auf. Der Grundkörper ist in Form eines Zapfens gestaltet und an einem hierfür vorgesehenen Abschnitt eines Tibiaschnittblocks befestigbar. Die beiden Taster sind jeweils relativ zu dem Grundkörper und zueinander um eine gemeinsame Schwenkachse schwenkbeweglich an dem Grundkörper gelagert. Die beiden Taster sind jeweils zwischen einem ersten Ende und einem zweiten Ende längserstreckt. Die ersten Enden sind jeweils zur Kontaktierung der proximalen Tibia, genauer: des Tibiaplateaus, eingerichtet. Bei dem bekannten chirurgischen Instrument sind die beiden Taster entlang der gemeinsamen Schwenkachse axial übereinander in unterschiedlichen Ebenen angeordnet. Zudem sind die beiden Taster in Längsrichtung überkreuzt angeordnet.

Aus der US 2019/0231365 A1 ist ein weiteres chirurgisches Instrument zur Ausrichtung des Tibiaschnitts bekannt. Auch dieses bekannte chirurgische Instrument weist einen zapfenförmigen Grundkörper und zwei Taster auf. Die beiden Taster sind wiederum um eine gemeinsame Schwenkachse schwenkbeweglich an dem zapfenförmigen Grundkörper gelagert, axial übereinander angeordnet sowie in Längsrichtung überkreuzt.

Aufgabe der Erfindung ist es, ein chirurgisches Instrument der eingangs genannten Art bereitzustellen, das Vorteile gegenüber dem Stand der Technik bietet und insbesondere eine verbesserte Ausrichtung des proximalen Tibiaschnitts ermöglicht.

Diese Aufgabe wird dadurch gelöst, dass der erste Taster relativ zu dem Grundkörper und dem zweiten Taster um eine erste Schwenkachse schwenkbeweglich an dem Grundkörper gelagert ist, und dass der zweite Taster relativ zu dem Grundkörper und dem erster Taster um eine zweite Schwenkachse schwenkbeweglich an dem Grundkörper gelagert ist. Die Erfindung geht von der Erkenntnis aus, dass eine gemeinsame Lagerung beider Taster an ein- und derselben Schwenkachse mit Nachteilen einhergehen kann. Denn durch die gemeinsame Lagerung an einer einzigen Schwenkachse sind die beiden Taster unmittelbar benachbart, übereinander und/oder überkreuzt angeordnet. Dies kann dazu führen, dass bei der Einstellung der Schwenkposition eines Tasters ungewollt eine bereits eingestellte Schwenkposition des weiteren Tasters verstellt wird. Bleibt ein solches Verstellen unbemerkt, kann dies zu einer fehlerhaften Ausrichtung des Tibiaschnitts führen. Die Erfindung wirkt dem entgegen. Zu diesem Zweck sind die beiden Taster voneinander unabhängig an jeweils einer separaten Schwenkachse an dem Grundkörper gelagert. Vorzugsweise sind die Taster in einer gemeinsamen Ebene längserstreckt. Vorzugsweise sind die Taster nicht überkreuzt angeordnet. Das erste Ende des ersten Tasters ist zur Kontaktierung eines medialen Abschnitts des Tibiaplateaus eingerichtet und das erste Ende des zweiten Tasters ist zur Kontaktierung eines lateralen Abschnitts des Tibiaplateaus eingerichtet oder umgekehrt. Die beiden Schwenkachsen sind bei einer Ausgestaltung jeweils als geometrische Achse (englisch: axis) aufzufassen. Bei einer weiteren Ausgestaltung sind die beiden Schwenkachsen alternativ oder zusätzlich jeweils als gegenständliche Achse (englisch: axle) im Sinne eines Abschnitts und/oder Bauteils des Grundkörpers aufzufassen. Vorzugsweise sind die beiden Schwenkachsen proximodistal orientiert.

Die in dieser Beschreibung verwendeten Lage- und Richtungsbezeichnungen beziehen sich auf den Körper eines Patienten, insbesondere dessen Tibia, und sind insoweit gemäß ihrer üblichen anatomischen Bedeutung zu verstehen. Folglich bedeutet "anterior" vordere(r) oder vorne liegend, "posterior" hintere(r) oder hinten liegend, "medial" innere(r) oder innen liegend, "lateral" äußere(r) oder außen liegend, "proximal" zum Körperzentrum hin und "distal" vom Körperzentrum entfernt. Weiter bedeuten "proximodistal" entlang, vorzugsweise parallel zu, einer proximal-distal ausgerichteten Achse, "anteroposterior" entlang, vorzugsweise parallel zu, einer anterior-posterior ausgerichteten Achse und "mediolateral" entlang, vorzugsweise parallel zu, einer medial-lateral ausgerichteten Achse. Die besagten Achsen sind orthogonal zueinander ausgerichtet und können selbstverständlich in Bezug zu nicht mit der Anatomie des Patienten in Verbindung stehenden X-, Y- und Z-Achsen gesetzt werden. Beispielsweise kann die proximaldistale Achse alternativ als X-Achse bezeichnet werden. Die medial-laterale Achse kann als Y-Achse bezeichnet werden. Die anterior-posteriore Achse kann als Z-Achse bezeichnet werden. Zur verbesserten Veranschaulichung und der Einfachheit der Bezeichnungen wegen werden nachfolgend in erster Linie die besagten anatomischen Lage- und Richtungsbezeichnungen verwendet. Weiter werden Bezeichnungen wie "Rückseite" einer Komponente oder eines Abschnitts des chirurgischen Instruments, beispielsweise des Grundkörpers, in Bezug auf eine distal gerichtete Blickrichtung verwendet. Dementgegen werden Bezeichnungen wie "Vorderseite" in Bezug auf eine proximal gerichtete Blickrichtung verwendet.

In Ausgestaltung der Erfindung sind die erste Schwenkachse und die zweite Schwenkachse um einen mediolateralen Abstand voneinander beabstandet an dem Grundkörper angeordnet. Die mediolaterale Beabstandung der beiden Schwenkachsen erlaubt eine verbesserte Positionierung der ersten Enden der beiden Taster. Vorzugsweise entspricht der mediolaterale Abstand einem üblichen Abstand zwischen dem medialen Tibiaplateau und dem lateralen Tibiaplateau. Hierdurch sind die beiden Taster bei einer entsprechenden Positionierung des jeweiligen ersten Endes an dem betreffenden Abschnitt des Tibiaplateaus in etwa parallel ausgerichtet. Der mediolaterale Abstand beträgt vorzugsweise zwischen 4 cm und 12 cm, bevorzugt zwischen 5 cm und 10 cm, besonders bevorzugt zwischen 6 cm und 8 cm.

In weiterer Ausgestaltung der Erfindung sind die erste Schwenkachse und die zweite Schwenkachse zueinander parallel. Eine solche parallele Ausrichtung bietet Vorteile. Bei weiteren Ausgestaltungen sind die Schwenkachsen zueinander nicht parallel orientiert.

In weiterer Ausgestaltung der Erfindung ist die erste Schwenkachse orthogonal zu der Längsachse des ersten Tasters und die zweite Schwenkachse ist orthogonal zu der Längsachse des zweiten Tasters. Die Längsachse des ersten Tasters wird nachfolgend auch als erste Längsachse bezeichnet. Die Längsachse des zweiten Tasters wird nachfolgend auch als zweite Längsachse bezeichnet. Eine solche orthogonale Ausrichtung der jeweiligen Schwenkachse in Bezug auf die jeweilige Längsachse hat sich als vorteilhaft erwiesen. Alternativ denkbar sind nicht orthogonale Ausrichtungen.

In weiterer Ausgestaltung der Erfindung sind die erste Schwenkachse und die zweite Schwenkachse in einer gemeinsamen ersten Ebene angeordnet, welche - in einem an dem Tibiaschnittblock befestigten Zustand des chirurgischen Instruments - orthogonal zu einer Schnittführungsebene des Tibiaschnittblocks ist. Die Schnittführungsebene ist diejenige Ebene, in welcher die Tibiaresektion mittels des Tibiaschnittblocks geführt anbringbar ist.

Beispielsweise weist der Tibiaschnittblock einen Aufnahmeschlitz auf, welcher zur Aufnahme und Führung eines Sägeblatts zum Anbringen des Resektionsschnitts eingerichtet ist. Der Aufnahmeschlitz definiert in diesem Fall die Schnittführungsebene. Alternativ oder zusätzlich kann der Tibiaschnittblock eine Anlagefläche zum Anlegen und Führen des Sägeblatts aufweisen. In diesem Fall definiert die Anlagefläche die Schnittführungsebene. In der Verwendung des Tibiaschnittblocks ist die Schnittführungsebene anteroposterior und mediolateral erstreckt und folglich eine Transversalebene. Die erste Ebene, in welcher die beiden Schwenkachsen angeordnet sind, ist parallel zu der Schnittführungsebene und/oder eine Frontalebene.

In weiterer Ausgestaltung der Erfindung ist der erste Taster relativ zu dem Grundkörper und zu dem zweiten Taster entlang einer ersten Führungsachse linearbeweglich an dem Grundkörper gelagert, und der zweite Taster ist relativ zu dem Grundkörper und dem ersten Taster entlang einer zweiten Führungsachse linearbeweglich an dem Grundkörper gelagert. Bei dieser Ausgestaltung der Erfindung sind beide Taster jeweils - zusätzlich zu der jeweiligen Schwenkbeweglichkeit - linearbeweglich. Zusätzlich zu der Einstellbarkeit der Schwenkposition kann folglich auch eine Linearposition eingestellt werden. Mit anderen Worten lassen sich die beiden Taster unabhängig voneinander entlang ihrer jeweiligen Längsachse verschieben. Die Längsverschieblichkeit der Taster erlaubt insbesondere die Verwendung an unterschiedlich großen Tibiaknochen. Die beiden Führungsachsen sind bei einer Ausgestaltung Achsen im geometrischen Sinne. Bei einer weiteren Ausgestaltung sind die beiden Führungsachsen alternativ oder zusätzlich gegenständliche Achsen.

In weiterer Ausgestaltung der Erfindung ist die erste Führungsachse parallel zu der Längsachse des ersten Tasters und die zweite Führungsachse ist parallel zu der Längsachse des zweiten Tasters. Eine solche parallele Ausrichtung der jeweiligen Führungsachse in Bezug auf die jeweilige Längsachse ist vorteilhaft, aber nicht zwingend. Dementsprechend sind die Führungsachsen bei weiteren Ausgestaltungen jeweils nicht parallel zu der jeweiligen Längsachse.

In weiterer Ausgestaltung der Erfindung sind die erste Führungsachse und die zweite Führungsachse in einer gemeinsamen zweiten Ebene angeordnet, welche - in einem an dem Tibiaschnittblock befestigten Zustand des chirurgischen Instruments - parallel zu einer Schnittführungsebene des Tibiaschnittblocks ist. Bezüglich der Schnittführungsebene des Tibiaschnittblocks gilt das bereits Gesagte. In der Verwendung des chirurgischen Instruments ist die zweite Ebene eine Transversalebene. Sofern das chirurgische Instrument eine erste Ebene aufweist, in welcher beide Schwenkachsen gemeinsam angeordnet sind, ist die zweite Ebene orthogonal zu der ersten Ebene ausgerichtet. Die parallele Orientierung der zweiten Ebene gegenüber der Schnittführungsebene geht mit Vorteilen einher. Bei weiteren Ausgestaltungen ist eine nicht parallele Ausrichtung vorgesehen.

In weiterer Ausgestaltung der Erfindung weist der Grundkörper einen ersten Lagerzapfen und einen zweiten Lagerzapfen auf, der erste Taster weist ein erstes Langloch auf, in welches der erste Lagerzapfen um die erste Schwenkachse und entlang einer Längserstreckungsrichtung des ersten Langlochs relativbeweglich eingreift, und der zweite Taster weist ein zweites Langloch auf, in welches der zweite Lagerzapfen um die zweite Schwenkachse und entlang einer Längserstreckungsrichtung des zweiten Langlochs relativbeweglich eingreift. Dies ist eine besonders bevorzugte Ausgestaltung der Erfindung. Der erste Lagerzapfen ist koaxial zu der ersten Schwenkachse ausgerichtet. Der zweite Lagerzapfen ist koaxial zu der zweiten Schwenkachse ausgerichtet. Mit anderen Worten definiert eine jeweilige Längserstreckung der beiden Lagerzapfen die jeweilige Schwenkachse. Das erste Langloch ist parallel zu der ersten Schwenkachse in den ersten Taster eingebracht. Das zweite Langloch ist parallel zu der zweiten Schwenkachse in den zweiten Taster eingebracht. Die Längserstreckungsrichtung des ersten Langlochs ist orthogonal zu der ersten Schwenkachse. Die Längserstreckungsrichtung des zweiten Langlochs ist orthogonal zu der zweiten Schwenkachse. Die Längserstreckungsrichtung des ersten Langlochs definiert eine/die erste Führungsachse. Die Längserstreckungsrichtung des zweiten Langlochs definiert eine/die zweite Führungsachse. Diese Ausgestaltung erlaubt eine konstruktiv einfache, präzise sowie robuste schwenkbewegliche und linearbewegliche Lagerung der beiden Taster an dem Grundkörper.

In weiterer Ausgestaltung der Erfindung weisen der erste Lagerzapfen und der zweite Lagerzapfen jeweils zwei axial beabstandete Radialbünde auf, zwischen welchen der jeweilige Taster axial formschlüssig gehalten ist. Die beiden Radialbünde des ersten Lagerzapfens wirken einer ungewollten axialen Verlagerung des ersten Tasters entgegen. Entsprechendes gilt mutatis mutandis für die beiden Radialbünde des zweiten Lagerzapfens. Die beiden Radialbünde des ersten Lagerzapfens sind entlang der ersten Schwenkachse voneinander beabstandet. Die beiden Radialbünde des zweiten Lagerzapfens sind entlang der zweiten Schwenkachse voneinander beabstandet.

In weiterer Ausgestaltung der Erfindung ist wenigstens einer der zwei Radialbünde rotationsunsymmetrisch und orthogonal zu der jeweiligen Schwenkachse länglich. Die insoweit rotationsunsymmetrische und längliche Gestaltung wirkt einer Kippbewegung des jeweiligen Tasters entgegen. Hierdurch wird eine besonders präzise Positionierung des Tasters, genauer: dessen ersten Endes, ermöglicht.

In weiterer Ausgestaltung der Erfindung ist eine mit dem ersten Taster und dem Grundkörper wirkverbundene erste Fixiereinrichtung vorhanden und zur lösbaren Fixierung der Schwenkbeweglichkeit und/oder der Linearbeweglichkeit des ersten Tasters eingerichtet, und es ist eine mit dem zweiten Taster und mit dem Grundkörper wirkverbundene zweite Fixiereinrichtung vorhanden und zur lösbaren Fixierung der Schwenkbeweglichkeit und/oder der Linearbeweglichkeit des zweiten Tasters eingerichtet. Die beiden Fixiereinrichtungen erlauben eine voneinander unabhängige lösbare Fixierung der beiden Taster. Dies im Gegensatz zu aus dem Stand der Technik bekannten Lösungen. Die beiden Fixiereinrichtungen wirken einem ungewollten Verstellen einer bereits vorgenommenen Positionierung der beiden Taster entgegen. Die beiden Fixiereinrichtungen sind bei unterschiedlichen Ausgestaltungen unterschiedlich ausgeführt, beispielsweise als Schraub-, Klemm-, Rast- und/oder Schnappmechanismus. Die erste Fixiereinrichtung bewirkt eine kraft- und/oder formschlüssige lösbare Fixierung des ersten Tasters relativ zu dem Grundkörper. Entsprechendes gilt mutatis mutandis für die zweite Fixiereinrichtung.

In weiterer Ausgestaltung der Erfindung weist der Grundkörper einen Plattenabschnitt mit einer eben erstreckten Rückseite auf, welche - in einem an dem Tibiaschnittblock befestigen Zustand des chirurgischen Instruments - parallel zu einer Schnittführungsebene des Tibiaschnittblocks ist. Bei dieser Ausgestaltung der Erfindung weist der Grundkörper eine plattenförmige Gestalt und/oder Plattenform auf. In der Verwendung des chirurgischen Instruments ist der Plattenabschnitt mediolateral und anteroposterior erstreckt. Die Rückseite ist proximal orientiert, d.h. eine Normalenrichtung der Rückseite weist in proximale Richtung. Die Rückseite ist eben und parallel zu der Schnittführungsebene. Bezüglich der Schnittführungsebene des Tibiaschnittblocks gilt das bereits Gesagte. Die insoweit eben und parallel erstreckte Rückseite erlaubt eine einfache visuelle Kontrolle der Ausrichtung des chirurgischen Instruments. Vorzugsweise ist die Rückseite auf einem proximodistalen Niveau angeordnet, welches dem Niveau der späteren Kondylenlinie der zu implantierenden Tibiakomponente entspricht. Vorzugsweise ist ein proximaler Abstand der Rückseite von der Schnittführungsebene entsprechend angepasst. Der besagte Abstand beträgt vorzugsweise zwischen X1 mm und X2 mm, bevorzugt zwischen X3 mm und X4 mm, besonders bevorzugt zwischen X5 mm und X6 mm.

In weiterer Ausgestaltung der Erfindung weist der Grundkörper einen federbelasteten Rasthebel auf, welcher - in einem an dem Tibiaschnittblock befestigten Zustand des chirurgischen Instruments - lösbar mit einem Rastabschnitt des Tibiaschnittblocks verrastet ist. Diese Ausgestaltung der Erfindung erlaubt eine besonders einfache und zuverlässige lösbare Befestigung des chirurgischen Instruments an dem Tibiaschnittblock. Der Rasthebel ist zur manuellen Betätigung eingerichtet. Vorzugsweise ist der Rasthebel auf einer Vorderseite des Plattenabschnitts angeordnet, welcher der Rückseite des Plattenabschnitts distal gegenüberliegt. Vorzugsweise ist der Rasthebel relativ zu dem Plattenabschnitt verlagerbar zwischen einer Befestigungsstellung und einer Freigabestellung. In der Befestigungsstellung ist der Rasthebel lösbar mit dem Rastabschnitt verrastet. In der Freigabestellung ist die besagte Verrastung freigegeben, so dass der Grundkörper von dem Tibiaschnittblock abgenommen werden kann.

Die Erfindung betrifft zudem ein chirurgisches Instrumentensystem zur Verwendung bei einer Kniegelenkersatzoperation mit einem chirurgischen Instrument nach einem der vorhergehenden Ansprüche und mit einem Tibiaschnittblock, an welchem das chirurgische Instrument lösbar befestigt ist. Bei einer Ausgestaltung weist das chirurgische Instrumentensystem zudem einen extramedullären Ausrichtstab auf, welcher lösbar an einem hierfür vorgesehenen Abschnitt des Tibiaschnittblocks befestigt ist.

Weitere Vorteile und Merkmale der Erfindung ergeben sich aus den Ansprüchen sowie aus der Beschreibung bevorzugter Ausführungsbeispiele der Erfindung, die anhand der Zeichnungen dargestellt sind.
- Fig. 1: zeigt in schematischer Perspektivdarstellung eine Ausführungsform eines erfindungsgemäßen chirurgischen Instrumentensystems mit einer Ausführungsform eines erfindungsgemäßen chirurgischen Instruments,
- Fig. 2: in schematischer Perspektivdarstellung eine intraoperative Situation, in welcher das chirurgische Instrumentensystem nach Fig. 1 an einer proximalen Tibia ausgerichtet ist,
- Fig. 3: eine weitere schematische Perspektivdarstellung der intraoperativen Situation nach Fig. 2,
- Fig. 4: das chirurgische Instrumentensystem nach den Fig. 1 bis 3 in einer schematischen Aufsicht,
- Fig. 5: eine schematische Schnittdarstellung entlang einer Schnittlinie A-A gemäß Fig. 4 und
- Fig. 6: eine schematische Perspektivdarstellung einer weiteren Ausführungsform eines erfindungsgemäßen chirurgischen Instrumentensystems.

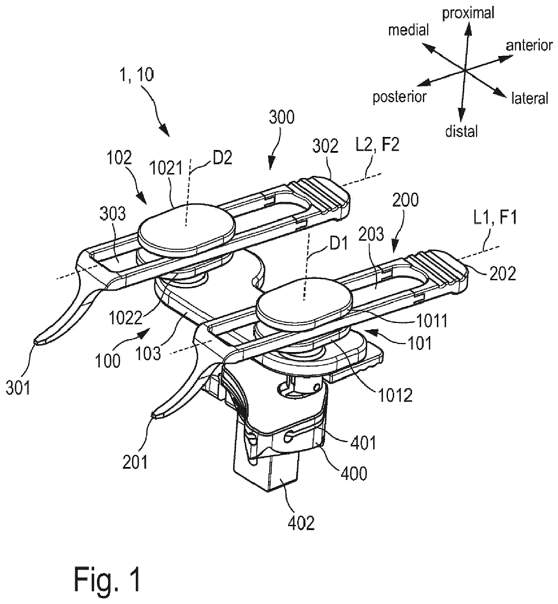

Gemäß den Fig. 1 bis 5 ist ein chirurgisches Instrument 1 zur Verwendung bei einer Kniegelenkersatzoperation vorgesehen und weist einen Grundkörper 100, einen ersten Taster 200 und einen zweiten Taster 300 auf.

Das chirurgische Instrument 1 ist auf noch näher beschriebene Weise an einem Tibiaschnittblock 400 lösbar befestigt. An dem Tibiaschnittblock 400 ist ein extramedullärer Ausrichtstab 600 lösbar befestigt. Zusammen mit dem Tibiaschnittblock 400 und dem extramedullären Ausrichtstab 600 bildet das chirurgische Instrument 1 ein chirurgisches Instrumentensystem 10.

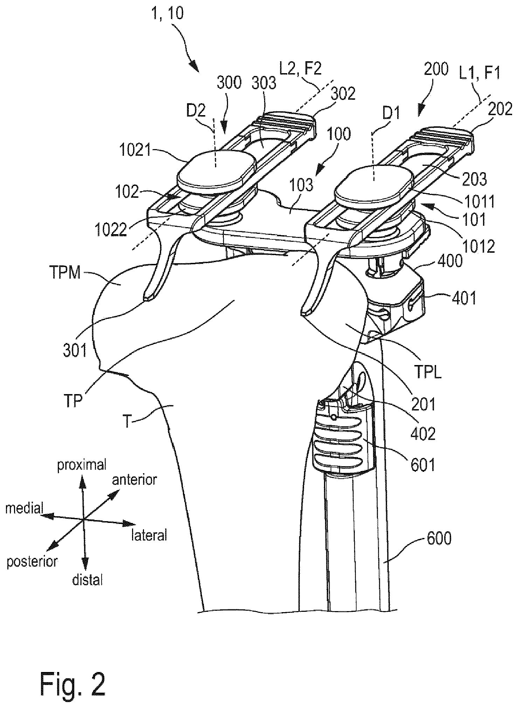

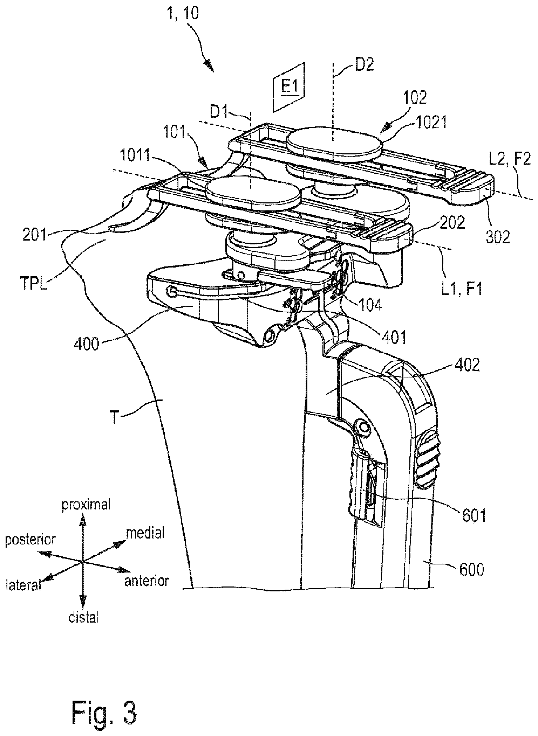

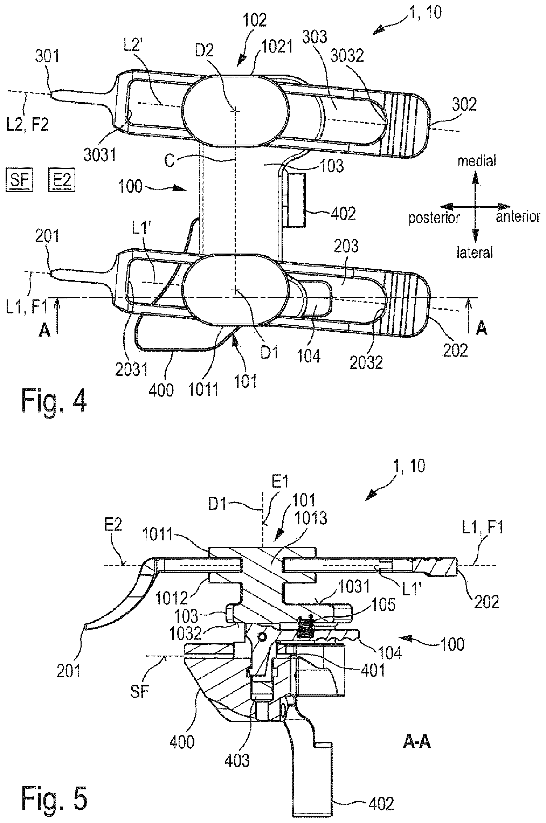

Der Tibiaschnittblock 400 ist auf eine dem Fachmann bekannte Weise zur Schnittführung an einer proximalen Tibia T eingerichtet (siehe Fig. 2, 3). Zu diesem Zweck weist der Tibiaschnittblock 400 einen Aufnahmeschlitz 401 auf, welcher zur Aufnahme und Führung eines Sägeblatts zur Resektion der proximalen Tibia T eingerichtet ist. Der Aufnahmeschlitz 401 definiert eine Schnittführungsebene SF (siehe Fig. 4, 5), in welcher der besagte Resektionsschnitt mittels des Tibiaschnittblocks 400 geführt ist. Der Tibiaschnittblock 400 weist zudem einen Befestigungsabschnitt 402 auf. Dieser ist auf eine dem Fachmann bekannte Weise zur lösbaren Befestigung an dem extramedullären Ausrichtstab 600 eingerichtet. Der extramedulläre Ausrichtstab 600 weist einen Befestigungsmechanismus 601 auf, welcher auf nicht näher gezeigte Weise mit dem Befestigungsabschnitt 402 zusammenwirkt. Der extramedulläre Ausrichtstab 600 dient einer Ausrichtung des Tibiaschnittblocks 400 in Bezug auf eine nicht näher bezeichnete anteriore Kante der Tibia T.

Das chirurgische Instrument 1 ist zur Ausrichtung des Tibiaschnittblocks 400 in Bezug auf anatomische Landmarken der proximalen Tibia T vorgesehen. Das chirurgische Instrument 1 kann auch als tibiales Ausrichtinstrument (englisch: tibial cut alignment device) bezeichnet werden. Bevor auf die spezifische Funktion des chirurgischen Instruments 1 näher eingegangen wird, wird zunächst dessen konstruktiver Aufbau weiter erläutert.

Der Grundkörper 100 ist auf noch näher beschriebene Weise zur lösbaren Befestigung an dem Tibiaschnittblock 400 eingerichtet.

Der erste Taster 200 und der zweite Taster 300 sind jeweils relativ zu dem Grundkörper 100 beweglich an demselben gelagert und weisen jeweils ein erstes Ende 201, 301 und ein zweites Ende 202, 302 auf. Der erste Taster 200 ist entlang einer ersten Längsachse L1 längserstreckt. Der zweite Taster 300 ist entlang einer zweiten Längsachse L2 längserstreckt. Die beiden Längsachsen L1, L2 definieren jeweils eine Haupterstreckungsrichtung des jeweiligen Tasters 200, 300. Die beiden ersten Enden 201, 301 sind jeweils zur Kontaktierung eines Tibiaplateaus TP der proximalen Tibia T eingerichtet (siehe Fig. 2, 3). Vorliegend ist das erste Ende 201 des ersten Tasters 200 zur Kontaktierung eines lateralen Abschnitts des Tibiaplateaus TP eingerichtet. Dieser Abschnitt des Tibiaplateaus TP wird nachfolgend als laterales Tibiaplateau TPL bezeichnet. Das erste Ende 301 des zweiten Tasters 300 ist zur Kontaktierung eines medialen Abschnitts des Tibiaplateaus TP eingerichtet. Dieser Abschnitt des Tibiaplateaus TP wird nachfolgend auch als mediales Tibiaplateau TPM bezeichnet. In Bezug auf die in den Figuren gezeigte intraoperative Situation können die Taster 200, 300 auch als lateraler Taster 200 und medialer Taster 300 bezeichnet werden.

Der erste Taster 200 ist relativ zu dem Grundkörper 100 und relativ zu dem zweiten Taster 300 um eine erste Schwenkachse D1 schwenkbeweglich an dem Grundkörper 100 gelagert. Der zweite Taster 300 ist relativ zu dem Grundkörper 100 und relativ zu dem ersten Taster 200 um eine zweite Schwenkachse D2 schwenkbeweglich an dem Grundkörper 100 gelagert. Die voneinander unabhängige und/oder gesonderte schwenkbewegliche Lagerung der beiden Taster 200, 300 erlaubt insbesondere eine verbesserte Positionierung der beiden ersten Enden 201, 301 an dem Tibiaplateau TP. Die verbesserte Positionierung geht mit einer besonders präzisen Ausrichtung des Tibiaschnittblocks 400 und damit auch der Schnittführungsebene SF einher.

In der Verwendung des chirurgischen Instruments 1 sind die beiden Schwenkachsen D1, D2 proximodistal ausgerichtet. Die Schnittführungsebene SF ist mediolateral und anteroposterior erstreckt.

Bei der gezeigten Ausführungsform sind die erste Schwenkachse D1 und die zweite Schwenkachse D2 zueinander parallel. Zudem sind die erste Schwenkachse D1 und die zweite Schwenkachse D2 um einen mediolateralen Abstand C voneinander beabstandet an dem Grundkörper 100 angeordnet (siehe Fig. 4). Bei der gezeigten Ausführungsform entspricht der mediolaterale Abstand C einem mediolateralen Abstand (ohne Bezugszeichen) zwischen dem medialen Tibiaplateau TPM und dem lateralen Tibiaplateau TPL. Bei einer anforderungsgerechten Kontaktierung der beiden ersten Enden 201, 301 an dem jeweiligen Abschnitt des Tibiaplateaus TP sind die beiden Taster 200, 300 somit wenigstens im Wesentlichen parallel zueinander längserstreckt.

Bei der gezeigten Ausführungsform ist die erste Schwenkachse D1 orthogonal zu der ersten Längsachse L1. Die zweite Schwenkachse D2 ist orthogonal zu der zweiten Längsachse L2.

Bei der gezeigten Ausführungsform sind die erste Schwenkachse D1 und die zweite Schwenkachse D2 in einer gemeinsamen ersten Ebene E1 angeordnet (siehe Fig. 3, 5). Die erste Ebene E1 ist orthogonal zu der Schnittführungsebene SF. Hierdurch bewegen sich die beiden ersten Enden 201, 301 bei einer Schwenkbewegung des jeweiligen Tasters 200, 300 jeweils in einem gleichbleibenden Abstand von der Schnittführungsebene SF.

Zusätzlich zu der besagten Schwenkbeweglichkeit der beiden Taster 200, 300 ist bei der gezeigten Ausführungsform eine Linearbewegung der beiden Taster 200, 300 möglich. Die Linearbeweglichkeit der beiden Taster 200, 300 relativ zu dem Grundkörper 100 und relativ zueinander ermöglicht eine nochmals verbesserte Positionierung der beiden ersten Enden 201, 301 an dem Tibiaplateau TP.

Vorliegend ist der erste Taster 200 relativ zu dem Grundkörper 100 und relativ zu dem zweiten Taster 300 entlang einer ersten Führungsachse F1 linearbeweglich an dem Grundkörper 100 gelagert. Der zweite Taster 300 ist relativ zu dem Grundkörper 100 und relativ zu dem ersten Taster 200 entlang einer zweiten Führungsachse F2 linearbeweglich an dem Grundkörper gelagert.

Bei der gezeigten Ausführungsform ist die erste Führungsachse F1 parallel zu der ersten Längsachse L1. Die zweite Führungsachse F2 ist parallel zu der zweiten Längsachse L2. Folglich ist die erste Führungsachse F1 orthogonal zu der ersten Schwenkachse D1 und die zweite Führungsachse F2 ist orthogonal zu der zweiten Schwenkachse D2. Zudem ist die erste Führungsachse F1 orthogonal zu der zweiten Schwenkachse D2. Die zweite Führungsachse F2 ist orthogonal zu der ersten Schwenkachse D1.

Die Orientierung der ersten Führungsachse F1 in Bezug auf den Grundkörper 100 und/oder die zweite Führungsachse F2 ist abhängig von der Schwenkstellung des ersten Tasters 200 in Bezug auf die erste Schwenkachse D1. Entsprechendes gilt mutatis mutandis für die Orientierung der zweiten Führungsachse F2.

Die erste Führungsachse F1 und die zweite Führungsachse F2 sind in einer gemeinsamen zweiten Ebene E2 angeordnet (siehe Fig. 4, 5). Die zweite Ebene E2 ist parallel zu der Schnittführungsebene SF. In der Verwendung des chirurgischen Instruments 1 ist die zweite Ebene E2 somit anteroposterior sowie mediolateral erstreckt. Die besagte parallele Orientierung der zweiten Ebene E2 gewährleistet, dass die beiden ersten Enden 201, 301 bei einer Linearbewegung des jeweiligen Tasters 200, 300 in einem unveränderlichen Abstand gegenüber der Schwenkführungsebene SF verbleiben. Bei der gezeigten Ausführungsform sind nicht lediglich die beiden Führungsachsen F1, F2 in einer/der gemeinsamen zweiten Ebene E2 angeordnet. Vielmehr gilt dies auch für die beiden Taster 200, 300. Die beiden Taster 200, 300 sind zwar mediolateral nebeneinander, nicht jedoch proximodistal übereinander und/oder versetzt angeordnet und/oder nicht überkreuzt.

Bei unterschiedlichen Ausgestaltungen ist die schwenkbewegliche Lagerung der beiden Taster 200, 300 auf unterschiedliche Art konstruktiv umgesetzt. Entsprechendes gilt hinsichtlich der linearbeweglichen Lagerung.

Bei der gezeigten Ausführungsform weist der Grundkörper einen ersten Lagerzapfen 101 und einen zweiten Lagerzapfen 102 auf. Der erste Taster 200 ist schwenk- und linearbeweglich an dem ersten Zapfen 101 gelagert. Der zweite Taster 300 ist schwenk- und linearbeweglich an dem zweiten Lagerzapfen 102 gelagert. Zu diesem Zweck weist der erste Taster 200 ein erstes Langloch 203 auf. Der erste Lagerzapfen 101 greift um die erste Schwenkachse D1 und entlang einer Längserstreckungsrichtung L1' des ersten Langlochs 203 relativbeweglich in das erste Langloch 203 ein. Entsprechendes gilt sinngemäß in Bezug auf den zweiten Taster 300. Der zweite Taster 300 weist ein zweites Langloch 303 auf. Der zweite Lagerzapfen 102 greift um die zweite Schwenkachse D2 schwenkbeweglich und entlang einer Längserstreckungsrichtung L2` des zweiten Langlochs 303 relativbeweglich in das zweite Langloch 303 ein.

Der erste Lagerzapfen 101 ist entlang der ersten Schwenkachse D1 längserstreckt. Mit anderen Worten definiert eine Axialrichtung des ersten Lagerzapfens 101 die erste Schwenkachse D1. Entsprechendes gilt sinngemäß für den zweiten Lagerzapfen 102. Dessen Axialrichtung definiert die zweite Schwenkachse D2.

Die Längserstreckungsrichtung L1' des ersten Langlochs 203 definiert die erste Führungsachse F1. Die Längserstreckungsrichtung L2' des zweiten Langlochs 303 definiert die zweite Führungsachse F2.

Bei der gezeigten Ausführungsform ist die Längserstreckung L1' des ersten Langlochs 203 koaxial zu der ersten Längsachse L1. Die Längserstreckungsrichtung L2' des zweiten Langlochs 303 ist koaxial zu der zweiten Längsachse L2.

Die beiden Taster 200, 300 sind jeweils gleitbeweglich an dem betreffenden Lagerzapfen 101, 102 geführt.

Das erste Langloch 203 ist zwischen einem ersten Ende 2031 und einem zweiten Ende 2032 längserstreckt. Das erste Ende 2031 fungiert als Anschlag bei einer anterioren Relativverlagerung des ersten Tasters 200. Das zweite Ende 203 fungiert als Anschlag bei einer posterioren Relativverlagerung des ersten Tasters 200. Das zweite Langloch 303 ist zwischen einem ersten Ende 3031 und einem zweiten Ende 3032 längserstreckt. Bezüglich deren Funktion gilt das bereits Gesagte mutatis mutandis. Das erste Langloch 203 ist in Axialrichtung des ersten Lagerzapfens 101 und/oder entlang der ersten Schwenkachse D1 durchgängig durch den ersten Taster 200 erstreckt. Das erste Langloch 203 ist folglich ein Durchgangsloch. Entsprechendes gilt sinngemäß für das zweite Langloch 303.

Sowohl die beiden Taster 200, 300 als auch die beiden Lagerzapfen 101, 102 sind bei der gezeigten Ausführungsform jeweils zueinander identisch gestaltet. Weitere gegenständliche und funktionelle Merkmale der beiden Lagerzapfen 101, 102 werden daher nachfolgend in erster Linie unter Bezugnahme auf den ersten Lagerzapfen 101 erläutert. Das zu dem ersten Lagerzapfen 101 Gesagte gilt sinngemäß auch für den zweiten Lagerzapfen 102.

Der erste Lagerzapfen 101 weist einen ersten Radialbund 1011 und einen zweiten Radialbund 1012 auf. Die beiden Radialbünde 1011, 1012 sind entlang der ersten Schwenkachse D1 voneinander beabstandet. Der erste Taster 200 ist entlang der ersten Schwenkachse D1 formschlüssig zwischen den beiden Radialbünden 1011, 1012 gehalten. Zudem ist der erste Taster 200 um die erste Schwenkachse D1 und entlang der ersten Führungsachse F1 gleitbeweglich zwischen den beiden Radialbünden 1011, 1012 geführt. Die beiden Radialbünde 1011, 1012 sind zueinander parallel orientiert.

Der erste Lagerzapfen 101 weist zudem einen Mittenabschnitt 1013 auf. Der Mittenabschnitt 1013 ist entlang der ersten Schwenkachse D1 zwischen dem ersten Radialbund 1011 und dem zweiten Radialbund 1012 angeordnet. Der Mittenabschnitt 1013 weist einen rotationssymmetrischen Querschnitt auf. Der erste Taster 200 ist um die erste Schwenkachse D1 gleitbeweglich an dem Mittenabschnitt 1013 geführt. Zudem ist der erste Taster 200 in der zweiten Ebene E2 senkrecht zu der ersten Führungsachse F1 (translatorisch) formschlüssig an dem Mittenabschnitt 1013 gehalten.

Die beiden Radialbünde 1011, 1022 sind jeweils rotationsunsymmetrisch und länglich. Die Rotationsunsymmetrie bezieht sich auf die erste Schwenkachse D1. Die längliche Ausführung bezieht sich auf die in den Figuren eingezeichnete anteroposteriore Achse. Die rotationsunsymmetrische und/oder längliche Gestaltung der beiden Rotationsabschnitte 1011, 1012 bietet Vorteile im Hinblick auf die bewegliche Lagerung des ersten Tasters 200. Insbesondere wird einer ungewollten Kippbewegung des ersten Tasters 200 entgegengewirkt.

In Entsprechung zu dem ersten Lagerzapfen 101 weist der zweite Lagerzapfen 102 einen ersten Radialbund 1021 und einen zweiten Radialbund 1022 sowie einen Mittenabschnitt (ohne Bezugszeichen) auf. Für alles Weitere wird auf die Offenbarung zu dem ersten Lagerzapfen 101 verwiesen.

Der Grundkörper 100 weist bei der gezeigten Ausführungsform einen Plattenabschnitt 103 auf. Der Plattenabschnitt ist mediolateral und anteroposterior erstreckt. Der erste Lagerzapfen 101 und der zweite Lagerzapfen 102 ragen axial von dem Plattenabschnitt 103 auf. Der Plattenabschnitt 103 weist eine Rückseite 1031 auf. Die Lagerzapfen 101, 102 ragen im Speziellen von der Rückseite 1031 auf. Die Rückseite 1031 ist proximal orientiert, d.h. eine nicht näher bezeichnete Normalenrichtung der Rückseite 1031 weist in proximale Richtung. Der Plattenabschnitt 103 weist zudem eine der Rückseite 1031 distal gegenüberliegende Vorderseite 1032 auf. Die Rückseite 1031 ist bei der gezeigten Ausführungsform eben und parallel zu der Schnittführungsebene SF. Die besagte Ausrichtung der Rückseite 1031 erlaubt eine einfache visuelle Kontrolle der Ausrichtung der Schnittführungsebene SF.

Bei unterschiedlichen Ausgestaltungen ist die lösbare Befestigung des Grundkörpers an dem Tibiaschnittblock unterschiedlich ausgeführt. Denkbar sind grundsätzlich eine Rast-, Klemm-, Schraub-, Schnapp- und/oder sonstige lösbare form- und/oder kraftschlüssige Fügeverbindung.

Bei der gezeigten Ausführungsform weist der Grundkörper 100 einen Rasthebel 104 auf. Der Rasthebel 104 ist in der in den Figuren gezeigten Konfiguration lösbar mit einem Rastabschnitt 403 des Tibiaschnittblocks 400 verrastet. Der Rasthebel 104 ist relativ zu dem Plattenabschnitt 103 zwischen einer Raststellung und einer Freigabestellung verlagerbar. Die Raststellung ist insbesondere in Fig. 5 im Detail ersichtlich. In der Raststellung wirkt der Rasthebel 104 lösbar formschlüssig mit dem Rastabschnitt 403 zusammen. In der Freigabestellung (in den Figuren nicht dargestellt) ist der Rasthebel 104 unter Aufhebung der Rastverbindung relativ zu dem Plattenabschnitt 103 verlagert. Bei der gezeigten Ausführungsform ist der Rasthebel 104 um eine nicht näher bezeichnete Schwenkachse relativ zu dem Plattenabschnitt 103 schwenkbeweglich. Zudem ist der Rasthebel 104 mittels eines Federelements 105 in Richtung der Raststellung federbelastet. Das Federelement 105 wirkt einem ungewollten Lösen der Rastverbindung entgegen. Das Federelement 105 ist bei der gezeigten Ausführungsform eine Wendelfeder. Das Federelement 105 ist einends an dem Plattenabschnitt 103 und andernends an dem Rasthebel 104 abgestützt.

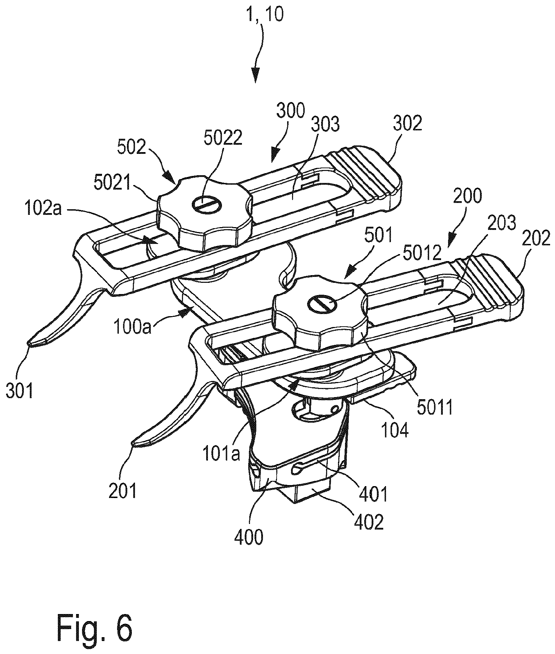

Fig. 6 zeigt eine weitere Ausführungsform eines erfindungsgemäßen chirurgischen Instruments 1a, welches zusammen mit dem Tibiaschnittblock 400 eine weitere Ausführungsform eines erfindungsgemäßen chirurgischen Instrumentensystems 10a bildet. Das chirurgische Instrumentensystem 10a kann zudem den/einen extramedullären Ausrichtstab 600 umfassen. Das chirurgische Instrument 1a ist hinsichtlich seines Aufbaus und seiner Funktion im Wesentlichen identisch mit dem chirurgischen Instrument 1 nach den Fig. 1 bis 5. Zur Vermeidung von Wiederholungen wird daher nachfolgend lediglich auf wesentliche Unterschiede des chirurgischen Instruments 1a gegenüber dem chirurgischen Instrument 1 nach den Fig. 1 bis 5 eingegangen. Funktionsgleiche Bauteile und/oder Abschnitte werden nicht gesondert erläutert. Stattdessen wird auf die Beschreibung des chirurgischen Instruments 1 verwiesen und ausdrücklich Bezug genommen.

Im Unterschied zu dem chirurgischen Instrument 1 nach den Fig. 1 bis 5 weist das chirurgische Instrument 1a eine erste Fixiereinrichtung 501 und eine zweite Fixiereinrichtung 502 auf. Die beiden Fixiereinrichtungen 501, 502 erlauben eine Fixierung der jeweiligen Schwenk- und Linearbeweglichkeit der beiden Taster 200, 300. Die beiden Fixiereinrichtungen 501, 502 sind hinsichtlich ihres Aufbaus und ihrer Funktion identisch. Zur Vermeidung von Wiederholungen wird daher zwecks Erläuterung weiterer Merkmale in erster Linie auf die erste Fixiereinrichtung 501 eingegangen. Das zu der ersten Fixiereinrichtung 501 Beschriebene gilt mutatis mutandis auch für die zweite Fixiereinrichtung 502.

Die erste Fixiereinrichtung 501 ist mit dem ersten Taster 200 und dem Grundkörper 100a wirkverbunden. Die erste Fixiereinrichtung 501 weist ein Feststellrad 5011 und einen Gewindeabschnitt 5012 auf. Das Feststellrad 5011 weist ein nicht näher bezeichnetes Innengewinde auf und ist gewindebeweglich mit dem Gewindeabschnitt 5012 verschraubt. Der Gewindeabschnitt 5012 ist einends des ersten Lagerzapfens des Grundkörpers 100a angeordnet. Das Feststellrad 5011 ist entlang der ersten Schwenkachse gewindebeweglich mit dem Gewindeabschnitt 5012 verschraubt. Zur Fixierung der Relativbeweglichkeit des ersten Tasters 200 wird das Feststellrad 5011 im Uhrzeigersinn festgeschraubt. Hierdurch drückt das Feststellrad 5011 auf eine nicht näher bezeichnete Rückseite des ersten Tasters 200. Der erste Taster 200 wird hierdurch zwischen dem Feststellrad 5012 und dem zweiten Radialbund (ohne Bezugszeichen) des Grundkörpers 100a festgeklemmt. Zum Lösen der Fixierung wird das Feststellrad 5011 entgegen dem Uhrzeigersinn betätigt.

Der Grundkörper 100a ist im Wesentlichen identisch zu dem Grundkörper 100 des chirurgischen Instruments 1 nach den Fig. 1 bis 5. Der wesentliche Unterschied liegt in der Gestaltung der beiden Lagerzapfen des Grundkörpers 100a. Im Unterschied zu den beiden Lagerzapfen 101, 102 weisen die Lagerzapfen 101a, 102a jeweils keinen ersten Radialbund auf. Stattdessen sind die Gewindeabschnitte 5012, 5022 vorgesehen. Vereinfacht ausgedrückt, treten die beiden Feststellräder 5011, 5021 anstelle des jeweiligen ersten Radialbunds.

## Patentansprüche

1. Chirurgisches Instrument (1, 1a) zur Verwendung bei einer Kniegelenkersatzoperation, aufweisend
einen Grundkörper (100, 100a), welcher eingerichtet ist zur lösbaren Befestigung an einem Tibiaschnittblock (400) zur Schnittführung an einer proximalen Tibia (T),
einen ersten Taster (200) und einen zweiten Taster (300), welche jeweils relativ zu dem Grundkörper (100, 100a) beweglich an demselben gelagert und jeweils entlang einer Längsachse (L1, L2) zwischen einem ersten Ende (201, 301) und einem zweiten Ende (202, 302) längserstreckt sind, wobei die ersten Enden (201, 301) jeweils zur Kontaktierung eines Tibiaplateaus (TP) der Tibia (T) eingerichtet sind,
**dadurch gekennzeichnet, dass** der erste Taster (200) relativ zu dem Grundkörper (100, 100a) und dem zweiten Taster (300) um eine erste Schwenkachse (D1) schwenkbeweglich an dem Grundkörper (100, 100a) gelagert ist, und dass der zweite Taster (300) relativ zu dem Grundkörper (100, 100a) und dem ersten Taster (200) um eine zweite Schwenkachse (D2) schwenkbeweglich an dem Grundkörper (100, 100a) gelagert ist.

2. Chirurgisches Instrument (1, 1a) nach Anspruch 1, **dadurch gekennzeichnet, dass** die erste Schwenkachse (D1) und die zweite Schwenkachse (D2) um einen mediolateralen Abstand (C) voneinander beabstandet an dem Grundkörper (100, 100a) angeordnet sind.

3. Chirurgisches Instrument (1, 1a) nach Anspruch 1 oder 2, **dadurch gekennzeichnet, dass** die erste Schwenkachse (D1) und die zweite Schwenkachse (D2) zueinander parallel sind.

4. Chirurgisches Instrument (1, 1a) nach einem der vorhergehenden Ansprüche, **dadurch gekennzeichnet, dass** die erste Schwenkachse (D1) orthogonal zu der Längsachse (L1) des ersten Tasters (200) ist, und dass die zweite Schwenkachse (D2) orthogonal zu der Längsachse (L2) des zweiten Tasters (300) ist.

5. Chirurgisches Instrument (1, 1a) nach einem der vorhergehenden Ansprüche, **dadurch gekennzeichnet, dass** die erste Schwenkachse (D1) und die zweite Schwenkachse (D2) in einer gemeinsamen ersten Ebene (E1) angeordnet sind, welche - in einem an dem Tibiaschnittblock (400) befestigten Zustand des chirurgischen Instruments (1, 1a) - orthogonal zu einer Schnittführungsebene (SF) des Tibiaschnittblocks (400) ist.

6. Chirurgisches Instrument (1, 1a) nach einem der vorhergehenden Ansprüche, **dadurch gekennzeichnet, dass** der erste Taster (200) relativ zu dem Grundkörper (100, 100a) und dem zweiten Taster (300) entlang einer ersten Führungsachse (F1) linearbeweglich an dem Grundkörper (100, 100a) gelagert ist, und dass der zweite Taster (300) relativ zu dem Grundkörper (100, 100a) und dem erster Taster (200) entlang einer zweiten Führungsachse (F2) linearbeweglich an dem Grundkörper (100, 100a) gelagert ist.

7. Chirurgisches Instrument (1, 1a) nach Anspruch 6, **dadurch gekennzeichnet, dass** die erste Führungsachse (F1) parallel zu der Längsachse (L1) des ersten Tasters (200) ist, und dass die zweite Führungsachse (F2) parallel zu der Längsachse (L2) des zweiten Tasters (300) ist.

8. Chirurgisches Instrument (1, 1a) nach Anspruch 6 oder 7, **dadurch gekennzeichnet, dass** die erste Führungsachse (F1) und die zweite Führungsachse (F2) in einer gemeinsamen zweiten Ebene (E2) angeordnet sind, welche - in einem an dem Tibiaschnittblock (400) befestigten Zustand des chirurgischen Instruments (1, 1a) - parallel zu einer Schnittführungsebene (SF) des Tibiaschnittblocks (400) ist.

9. Chirurgisches Instrument (1, 1a) nach einem der vorhergehenden Ansprüche, **dadurch gekennzeichnet, dass** der Grundkörper (100, 100a) einen ersten Lagerzapfen (101) und einen zweiten Lagerzapfen (102) aufweist, dass der erste Taster (200) ein erstes Langloch (203) aufweist, in welches der erste Lagerzapfen (101) um die erste Schwenkachse (D1) und entlang einer Längserstreckungsrichtung (L1') des ersten Langlochs (203) relativbeweglich eingreift, und dass der zweite Taster (300) ein zweites Langloch (303) aufweist, in welches der zweite Lagerzapfen (102) um die zweite Schwenkachse (D2) und entlang einer Längserstreckungsrichtung (L2') des zweiten Langlochs (303) relativbeweglich eingreift.

10. Chirurgisches Instrument (1) nach Anspruch 9, **dadurch gekennzeichnet, dass** der erste Lagerzapfen (101) und der zweite Lagerzapfen (102) jeweils zwei axial beabstandete Radialbünde (1011, 1012; 1021, 1022) aufweisen, zwischen welchen der jeweilige Taster (200, 300) axial formschlüssig gehalten ist.

11. Chirurgisches Instrument (1) nach Anspruch 10, **dadurch gekennzeichnet, dass** jeweils wenigstens einer der zwei Radialbünde (1011, 1012; 1021, 1022) rotationssymmetrisch und orthogonal zu der jeweiligen Schwenkachse (D1, D2) länglich ist.

12. Chirurgisches Instrument (1a) nach einem der vorhergehenden Ansprüche, **dadurch gekennzeichnet, dass** eine mit dem ersten Taster (200) und dem Grundkörper (100a) wirkverbundene erste Fixiereinrichtung (501) vorhanden und zur lösbaren Fixierung der Schwenkbeweglichkeit und/oder der Linearbeweglichkeit des ersten Tasters (200) eingerichtet ist, und dass eine mit dem zweiten Taster (300) und dem Grundkörper (100a) wirkverbundene zweite Fixiereinrichtung (502) vorhanden und zur lösbaren Fixierung der Schwenkbeweglichkeit und/oder der Linearbeweglichkeit des zweiten Tasters (300) eingerichtet ist.

13. Chirurgisches Instrument (1, 1a) nach einem der vorhergehenden Ansprüche, **dadurch gekennzeichnet, dass** der Grundkörper (100, 100a) einen Plattenabschnitt (103) mit einer eben erstreckten Rückseite (1031) aufweist, welche - in einem an dem Tibiaschnittblock (400) befestigten Zustand des chirurgischen Instruments (1, 1a) - parallel zu einer Schnittführungsebene (SF) des Tibiaschnittblocks (400) ist.

14. Chirurgisches Instrument (1, 1a) nach einem der vorhergehenden Ansprüche, **dadurch gekennzeichnet, dass** der Grundkörper (100, 100a) einen federkraftbelasteten Rasthebel (104) aufweist, welcher - in einem an dem Tibiaschnittblock (400) befestigten Zustand des chirurgischen Instruments (1, 1a) - lösbar formschlüssig mit einem Rastabschnitt (403) des Tibiaschnittblocks (400) verrastet ist.

15. Chirurgisches Instrumentensystem (10, 10a) zur Verwendung bei einer Kniegelenkersatzoperation mit einem chirurgischen Instrument (1, 1a) nach einem der vorhergehenden Ansprüche und mit einem Tibiaschnittblock (400), an welchem das chirurgische Instrument (1, 1a) lösbar befestigt ist.
